# EUROPEAN PATENT SPECIFICATION

(11) **EP 3 439 812 B1**
(45) Date of publication and mention of the grant of the patent: **14.08.2024**
(21) Application number: 17779429.4
(22) Date of filing: 04.04.2017
(51) Int. Cl.: B22D 2/00, G01N 11/04, B22C 9/00

(54) **DEVICE FOR MEASURING FLUIDITY OF MOLTEN METAL**
VORRICHTUNG ZUR MESSUNG DER FLIESSFÄHIGKEIT VON METALLSCHMELZE
DISPOSITIF DE MESURE DE LA FLUIDITÉ DE MÉTAL FONDU

(30) Priority: 05.04.2016 SE 1630073
(43) Date of publication of application: 13.02.2019
(73) Proprietor: Liljenfors, Tomas, 343 38 Älmhult (SE)
(72) Inventor: Liljenfors, Tomas, 343 38 Älmhult (SE)
(74) Representative: Industripatent i Växjö AB
(86) International application number: PCT/SE2017/000023
(87) International publication number: WO 2017/176184

(56) References cited:
- CN-A- 103 424 338
- CN-A- 103 424 338
- CN-B- 103 527 892
- CN-U- 202 118 158
- CN-U- 202 415 368
- US-A- 2 395 254
- US-A- 2 395 254
- US-A- 2 595 293
- US-A- 2 595 293
- US-A- 5 137 169
- US-A1- 2008 286 709
- US-A1- 2010 076 137

## Description

### Technical area of the invention

The present invention is related to a device for measuring fluidity of molten metal, wherein the device comprises a runway for molten metal.

### Background

In production of metal parts from molten metal, i.e. in the casting industry, fluid metal is used for production of functional gods or ingots. Casting process stability is influenced by several factors such as temperature of the molten metal, chemical composition and purity such as degree of contamination from non-metal contents. Temperature of the metal and chemical composition are parameters that are relatively easy to measure using conventional techniques. However reliable measurements of degree of contamination are more difficult.

Actually, the connection between castability and measurable quantities is complex. In reality it is difficult in casting industry to use the relation between castability and measurable quantities as a process tool. There exists a number of tools today for measuring fluidity of metal however common for all existing methods is that they are technically complicated thereby strongly operator dependent, and they are costly both by high purchase price and high operating cost. Thus, cast houses today often do not measure fluidity but instead rely on chemical composition and dissolved gas measurements only.

One of the most important parameters for casting of light metals, but also for other metals, are the amount of pollution of low weight substances such as oxides, which are difficult to measure during the casting process with the technologies known today.

US2395254 and US2595293 show runways for measurement of fluidity of molten metal where the distance that the molten metal flow before being solidified is used as a measure of fluidity of the metal. One problem with the runways of the kind shown in US2395254 and US2595293, which are made of sand, is that they need to have a relatively large cross section area in order to get a reliable result. If the cross-section area is too small the metal will be solidified within only a few seconds, or even less in time, which gives a very short distance to measure and thereby an uncertain result. Thus, runways of the kind shown in US2395254 and US2595293 should, if being able to provide reliable results, be quite large and thereby expensive to transport. It is therefore a need to provide a tool for measurement of fluidity for fluid metal, the tool being more efficient to transport and easier to handle at cast houses.

### Summary of the invention

The present invention is related to a device for measuring fluidity of molten metal, wherein the device comprises a runway for molten metal, wherein at least one surface of the runway comprises refractory fiber. Refractory fiber has lower heat conductivity that sand thereby it is possible to achieve more accurate measurements since the molten metal will be allow to flow a longer distance before solidifying in a runway comprising refractory fiber. Moreover, refractory fibre has a lower weight than sand and is therefore more costly to transport. Production of a runway in refractory fibre is more efficient since it is possible to use milling, and even possible to automatize production. The refractory fibre may be ceramic fibre, RCF (Refractory Ceramic Fiber), AES (Alkali Earth Silicate) Fibre or wool, glass fibre or wool or mineral wool, mineral fiber, stone wool, slag wool, mineral cotton, man-made mineral fibre (MMMF), man-made vitreous fiber (MMVF), high-temperature insulation wool (HTIW), ceramic fibre wool, Alumino silicate wool (ASW), Polycrystalline wool (PCW) or other applicable refractory fibre.

Refractory fibre can keep its form at temperatures of molten metals. The density of refractory fibre of below 1 kg/dm3 and refractory fibre has a coefficient of thermal conductivity below 1 W/mK, preferably below 0.5 W/mK. Low heat conductivity will allow the molten metal to flow a longer distance which will give more accurate results. Refractory fibre are available in crystalline, amorphous and/or non-crystalline appearance in different appearance as woven, non-woven, wool, short fibre (less than 100um), longer fibre more than 100um) with or without a binder binding the fibre together. The surface of the runway is considered to be a surface layer of at least 2 mm, which means that if the runway is provided with a thin coating, for instance 1 mm, it should still be considered as the surface of the runway comprising refractory fibre. One example of such surface coating is ceramic based mold coatings. The purpose of a surface coating may be to get a more even surface and / or get better heat resistance properties to enable the use at higher temperatures.

According to one embodiment the surface of the runway may be a lower surface of the runway, or an upper surface of the runway, or a side wall of the runway. In one embodiment, essentially the entire runway is made of refractory fibre.

According to one embodiment the runway comprises an upper portion and a lower portion, which may facilitate manufacturing of the device.

According to one embodiment the runway comprises an intermediate portion wherein side walls of the runway are formed by the intermediate portion. The intermediate portion may be of a different material than the upper and the lower portion. For example, the intermediate portion may be of metal and the upper and lower portions may comprise refractory fibre. For instance, the intermediate portion may comprise a metal tape.

According to one embodiment the device comprises an upper side of an upper portion wherein a filling position is defined by an inlet hole for positioning of a cup for filling molten metal into the runway though the inlet hole. A predefined filling position increases the chances of repeatability of measurements.

According to one embodiment the device comprises a cup for filling of molten metal into the runway. The cup may comprise refractory fibre.

According to one embodiment a bottom surface of the cup is formed by an upper portion of the upper side of the device. This may save material since the cup do not need to have a separate bottom surface. The upper side of the upper portion of the device may comprise a recess adapted to hold the cup. Thus, a well-defined position for the cup is given by the recess. The recess may also provide stability for the cup, and thereby allowing the cup to be made with relatively thin walls to save material. The cup may be formed as a frustum of a cone having side wall inclined downwards and inwards.

According to one embodiment the device comprises a plug for preventing molten metal to flow from the cup into the runway. The plug may comprise refractory fibre.

According to one embodiment the plug comprises a thermometer for measurement of the temperature of the molten metal in the cup. Since it is relevant to read the temperature at close to the inlet of the runway, the plug may be a suitable placement for a thermometer.

According to one embodiment the runway is closed and an exterior side of the device comprises a scale for reading essentially the distance of travel of the molten metal in the runway. The runway is constructed in a material, as refractory fibre or other materials that has been added to the refractory fibre, that change color upon contact with the liquid metal. As the material is affected by the liquid metal heat radiation, the material thereby changes color, and the readout can be observed on the upper side of the device as the color change mark the distance that the metal has traveled before solidifying. The change in color can also be obtained by a treatment of the upper side of the device being covered by a material or solution that changes the color by temperature. A marked scale on the upper part of the device is favorable for the operator as the device in this way do not have to be opened to read out the result.

According to one embodiment the device comprises a through hole for allowing molten metal to flow via the through hole and be collected in a container below the runway.

The through hole induces another fluidity measurement by measuring the time that a defined amount of liquid metal travel through the hole, into a container below the runway. The through hole could be placed close to the inlet of the runaway in a way that the liquid metal is divided into two individual paths, one flows into the runaway, one flows into the through hole. Two measurements can in this way be arranged for one sample.

According to one embodiment the container for collecting molten metal from the through hole may have a scale for direct reading of the amount of material in the container.

The present invention comprises a method of manufacturing a device for measurement of fluidity of molten metal, comprising: providing a board comprising refractory fibre; and forming a runway in the board. The runway may be formed by one of CNC milling or other types of milling, placing tape or nails to form the runway, cutting methods using water or laser, punching. There may also be other suitable ways of achieving the runway in the board.

The following may be serve for a divisional application: The runway as described above for measurement of fluidity of molten metal may comprise glass. In such a context, the runway is to be seen as a mold for metal molding wherein the mold comprise glass. By glass is preferably meant soda lime glass that originally is produced using the main ingredient of sand, soda and lime. It may be especially advantageously to use recycled glass. In that context, the mold should not be limited to a spiral shaped runway or even a runway at all. On the contrary, all types of suitable shapes and dimensions of a mold should be considered to fall within that scope. The term mold includes also cores. For example, an engine block may be casted using a mold for metal casting, where the mold comprises glass. To be able to use glass in a mold for metal casting, the glass preferably needs to be crushed and sorted by size by sieving the glass into appropriate fractions, as example the interval 0.1 micrometer to 2 millimeters, preferentially 50 micrometers to 500 micrometers. The sieved glass may be mixed to a binder as example water glass or other appropriate binder. The binding can also be performed by sintering the glass particles together.

### Brief description of the drawings

Fig. 1 is a perspective view of one embodiment of the present invention.
Fig. 2 shows the invention in Fig. 1 with the different parts arranged at a distance from each other.
Figs 3a-e show measurement steps for measurement of fluidity using the invention in Fig. 1.
Fig. 4 shows a second embodiment according to the invention in a perspective view and partly in cut though view.

### Detailed description of preferred embodiments

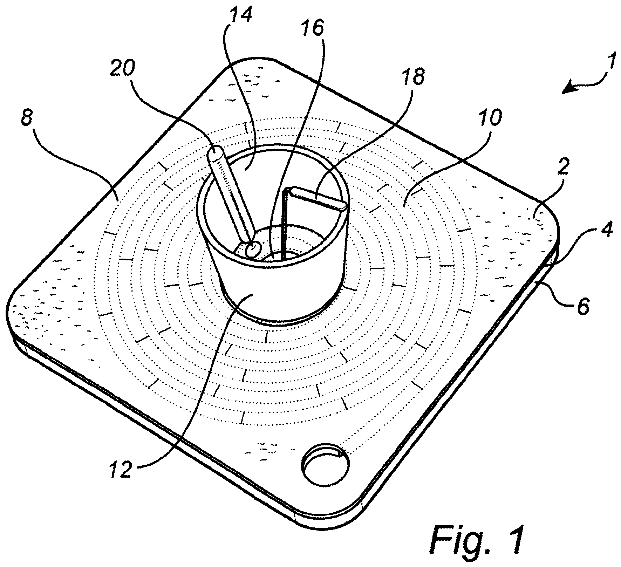

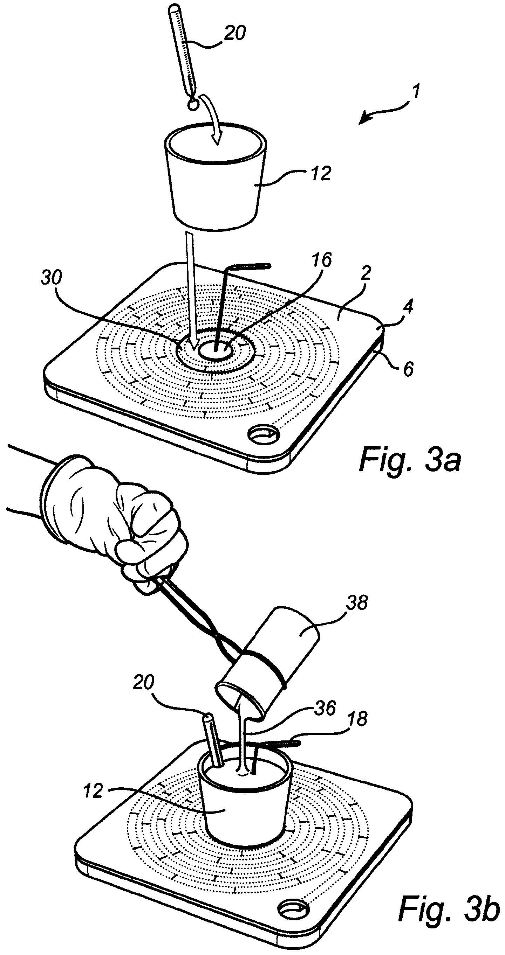

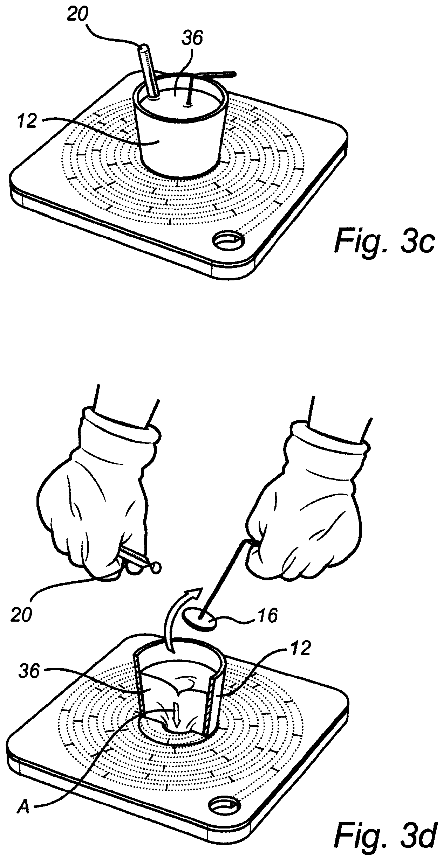

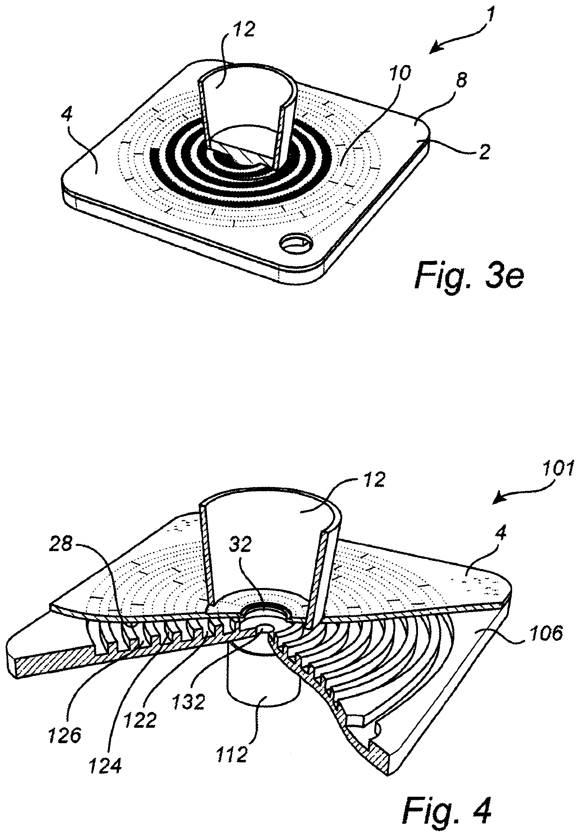

Fig. 1 shows a device 1 for measurement of fluidity of molten metal. The device 1 comprises an essentially square shaped board 2 made from one upper board part 4 and one lower board part 6. The upper side 8 of the upper board part 4 comprises a scale 10 for convenient reading of measurement result, which is described with reference to Fig. 3 e below.

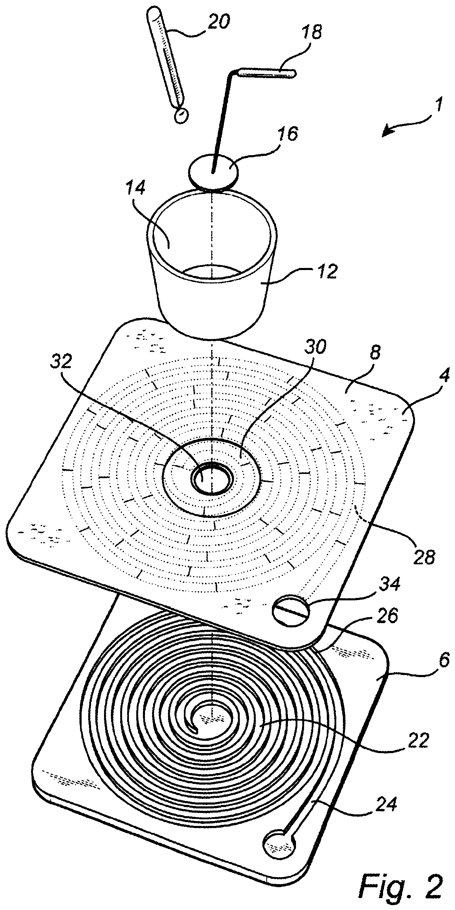

The device 1 further comprises a cup 12 being shaped as a frustum of a cone having walls 14 tapering inwards. The cup 12 do not have any bottom walls meaning that when the cup 12 is arranged on the upper board part 4, as is shown in Fig. 1, the upper board part 4 serve as a bottom for the cup 12. The device 1 further comprises a plug 16, which is described below with reference to Fig. 2, attached to a handle 18. A thermometer 20 is used for measuring the temperature of the molten metal poured into the cup 12 for use of the device 1.

The board 2, the cup 12 and the plug 16 are made from fibre board with fibre chosen from oxides of silicon, zirconium, magnesium, iron or calcium. There are examples of refractory fibre materials however a skilled person realizes that there are many different refractory fibre or mix of fibre that may be suitable such as mineral fibre, ceramic fibre, glass wool, carbon fibre.

Fig. 2 shows the device described regarding Fig. 1 above, having the parts arranged at a distance from each other to get a better understanding of the design of the device 1. The lower board part 6 comprises a spiral shaped runway 22. The runway 22 is formed by machining of the lower board part 6 in a milling machine to get the spiral shape runway 22. Since the lower board part 6 is made from refractory fibre all runway 22 walls, i.e. both lower parts 24 and side walls 26 of the runway 22, are made from refractory fibre. The lower side of the upper board part 4 will become the upper surface 28 of the runway 22, also these are made of refractory fibre since the entire upper board part 4 is made from refractory fibre. During use of the device 1 for measurements of fluidity of molten metal the upper board part 4 and the lower board part 6 are held together, for example by placing a weight on top of the upper board part 6 or by gluing the upper board part 4 and the lower board part 6 together. Thus, the runway 22 comprises lower surfaces 24, sidewalls 26 and upper surfaces 28.

A recess 30 for placement of the cup 12 is arranged in the center of the upper side 8 of the upper board part 4. Due to the recess 30 the cup 12 is kept stable during measurements and moreover the user easily realizes where to place the cup 12. In addition, the recess 30 provides some support to the sides of the cup 12, and the cup 12 does not need a bottom surface but instead the recess 30 forms the bottom of the cup 12.

The upper board part 4 comprises two through inlet holes 32, 34 - one placed in center of the upper board part 4 and another close to the outer edge of the upper board 4. The centrally placed inlet hole is intended as a primary inlet opening 32 and the inlet hole near the outer edge is an alternative inlet opening 34. The plug 16 is adapted to be place in the inlet opening 32 after the cup 12 is placed in the recess 30 in the upper board part 4. The plug 4 is attached to a handle for facilitating removal of the plug 12 when a measurement should start. The thermometer 20 should be placed in the cup 12 to measure the temperature of the molten metal thereby deciding when the measurement should start.

Figs 3a-e show step-by-step how the device 1 described above with reference to Fig. 1 and Fig. 2is used for measurement of fluidity of molten metal, such as aluminum. Molten aluminum is mentioned here as one example of metal for which the deceive 1 can be used to measure fluidity, however it is also possible to use the device 1 for measurements of fluidity for other metals and alloys such as for instance magnesium, copper, iron, alloys of the mentioned metals, etc.

Fig. 3a shows the upper and the lower board parts 4, 6, glued together to form one board 2, with the plug 16 arranged in the inlet hole 32 (see Fig. 2). The cup 12 should to be placed at the recess 30 of the board 2 and the thermometer 20 should be placed in the cup 12. Thereafter the device 1 is ready to be used for measurement for fluidity of molten metal.

Fig. 3b shows molten aluminum 36 poured into the cup 12 using a supply cup 38. The handle 18 of the plug is arranged such that it is sticking out from the cup 12.

Fig. 3c shows molten aluminum 36, which has been poured into the cup 12, and the thermometer 20 indicating that it is time to withdraw the plug. For molten aluminum, a temperature of for example 700 degrees Celsius, for the molten metal arranged close to the inlet hole, may be a relevant temperature to start the measurement. The temperature may be higher or lower when the measurement starts. The cup is preferably provided with a marking (not shown) showing the how much molten metal that should be poured into the cup. Alternatively, molten metal may be filled up to an edge of the cup (not shown).

Fig. 3d shows that the temperature of the molten aluminum 36 in the cup 12 has decreased to a suitable, predefined, level whereby the measurement can start by removing the plug 16. The thermometer 20 should be removed. Molten aluminum 36 flow through the inlet hole 32 which is illustrated by the arrow A.

Fig. 3e shows that the metal, here the aluminum, is solidified in the spiral shaped runway 22 (see Fig. 2) of the device 1 and in the cup 12. Since the runway 22 is arranged inside the board 2 the spiral shaped solidified metal is not visible from the outside. However, since the upper board part 4 has a relatively small thickness, for instance 1 to 20 mm, preferably 2-10 mm, most preferably 6 mm, the upper board part 4 will be colored throughout the board from the heat given from the molten metal. The scale 10 on the upper side of the board 2 thus shows the length of the aluminum spiral, thereby can be read as a measurement of fluidity of the molten aluminum.

Fig. 4 shows an alternative embodiment of the present invention. The cup 12 and the upper board 4 is of the same kind as was described above with reference to Fig. 1. The lower board part 106 has a runway 122 in the same sense as was described for the lower board part 6 with reference to Fig. 2 above. In this embodiment, however the lower board part 106 comprises a through hole 132 arranged straight below the inlet hole 32 of the upper board part 4. A container 112 is placed below the through hole 132 of the lower board part 106.

Fluidity measurement using the device 101 of the second embodiment will allow molten metal to flow from the cup 12 and into the runway 122 in the same manner as for the first embodiment. However, one portion of the molten metal will, instead of flowing into the runway, flow via the through hole 132 into the container 112. Thus, with this second embodiment two different fluidity measurements are made in the same test, which may give a more accurate total test result. The container interior walls may be provided with a scale (not shown) to easily be able to read the amount of metal provided in the container. A third fluidity measurement can be measured with this device since the time from start of molten metal into the hole 132 to end of flow, for instance using an optic device.

Fig. 4 shows the board parts 4, 106 in cross section showing the surfaces' of the runway 122, i.e. the lower surface 124, the side wall 126, and the upper surface 28. The runway 22 described above with reference to Fig. 1 has corresponding surfaces.

As has been described above the runway can be partly made from refractory fibre and partly from another material, for instance the side walls or other parts of the runway can be made of metal. However, it is possible that the entire runway is made of refractory fibre, or that at least 80% of the runway is made of refractory metal.

The cup may be of different heights depending on the molten metal that is intended to be used for the measurements. For instance, if measurements need to be done at a relatively low temperature of the molten metal the cup may need to have a larger height in order to allow the cup to be filled with a larger volume of molten metal thus giving a larger pressure on the molten metal that flow into the runway. Therefore, cups of different heights may be provided and suitable to use with a standard runway.

The cup may be provided with some sort of tip-over protection to prevent the cup from tipping when it is filled with molten metal. For instance a metal ring, with a diameter corresponding to the diameter of the cup, may be arranged on or attach to the cup to enhance stability of the cup. Further the metal ring could provide even better stability to the cup if the ring is attached to e.g. three legs or feet which are supported on the upper portion of the device. The ring is described as a metal ring but could be of other materials or shape, such as carbon fiber clamp or a ceramic material cup.

The invention has been described with references to a number of embodiments, however they can be varied in numerous way within the frames of the claims. For instance, the two board parts may be held together by other means than being glued together. A weigh or a clamp may be used, or fixing agents already present in the board material may be used.

The runway 22, 122 has inhere been described as being delimited by an upper board part 4 and a lower board part 6, 106, and that the runway is milled into the lower board part 6, 106. A person skilled in the art realized however that the runway may be milled in the upper board part, or in both board parts. It may even be possible to use several more board parts to define the runway. Another possibility is to use metal tape or metal ribbon between the upper and lower board to form the runway.

## Claims

1. Device (1) for measuring fluidity of molten metal (36), wherein said device (1) comprises a runway (22) for molten metal (36), and an upper side of the device comprises an inlet hole for filling molten metal into the runway, **characterized in that** the runway (22) comprises refractory fibre.

2. Device (1) according to claim 1, wherein the runway (22) comprises an upper portion (4) and a lower portion (6, 106).

3. Device (1) according to claim 2, wherein the runway (22) comprises an intermediate portion wherein side walls (26, 126) of the runway (22, 122) are formed by the intermediate portion.

4. Device (1) according to anyone of the previous claims, comprising a cup (12) for filling of molten metal into the runway (22).

5. Device (1) according to claim 4, wherein the cup (12) comprises refractory fibre.

6. Device (1) according to anyone of claims 4-5, wherein a bottom surface of the cup (12) is formed by an upper portion (4) of the upper side (8) of the device (1).

7. Device (1) according to anyone of claims 4-6, wherein the device (1) comprises a plug (16) for preventing molten metal to flow from the cup (12) into the runway (22).

8. Device (1) according to claim 7, wherein the plug (16) comprises a thermometer for measurement of the temperature of the molten metal in the cup (12).

9. Device (1) according to anyone of the previous claims, wherein the runway (22) is closed and an exterior side of the device (1) comprises a scale for reading the distance of travel of the molten metal (36) in the runway (22).

10. Device (101) according to anyone of the previous claims, wherein the device comprises a through hole (132) for allowing molten metal to flow via the through hole (132) and be collected in a container (112) below the runway (22).

11. Use of a device (1) for measuring fluidity of molten metal (36), wherein said device (1) comprises a runway (22) for molten metal (36), **characterized in that** the runway (22) comprises refractory fibre.

## Patentansprüche

1. Vorrichtung (1) zum Messen der Fließfähigkeit von Metallschmelze (36), wobei die Vorrichtung (1) einen Gießlauf (22) für Metallschmelze (36) und eine Oberseite der Vorrichtung ein Einlaufloch zum Einfüllen von Metallschmelze in den Gießlauf umfasst, **dadurch gekennzeichnet, dass** der Gießlauf (22) feuerfeste Fasern umfasst.

2. Vorrichtung (1) nach Anspruch 1, wobei der Gießlauf (22) einen oberen Abschnitt (4) und einen unteren Abschnitt (6, 106) umfasst.

3. Vorrichtung (1) nach Anspruch 2, wobei der Gießlauf (22) einen Zwischenabschnitt umfasst, wobei Seitenwände (26, 126) des Gießlaufs (22, 122) von dem Zwischenabschnitt gebildet werden.

4. Vorrichtung (1) nach einem der vorhergehenden Ansprüche, die einen Becher (12) zum Einfüllen von Metallschmelze in den Gießlauf (22) umfasst.

5. Vorrichtung (1) nach Anspruch 4, wobei der Becher (12) feuerfeste Fasern umfasst.

6. Vorrichtung (1) nach einem der Ansprüche 4-5, wobei eine Bodenfläche des Bechers (12) von einem oberen Abschnitt (4) der Oberseite (8) der Vorrichtung (1) gebildet wird.

7. Vorrichtung (1) nach einem der Ansprüche 4-6, wobei die Vorrichtung (1) einen Stopfen (16) umfasst, der verhindert, dass Metallschmelze aus dem Becher (12) in den Gießlauf (22) fließt.

8. Vorrichtung (1) nach Anspruch 7, wobei der Stopfen (16) ein Thermometer zum Messen der Temperatur der Metallschmelze in dem Becher (12) umfasst.

9. Vorrichtung (1) nach einem der vorhergehenden Ansprüche, wobei der Gießlauf (22) geschlossen ist und eine Außenseite der Vorrichtung (1) eine Skala zum Ablesen des von der Metallschmelze (36) in dem Gießlauf (22) zurückgelegten Wegs umfasst.

10. Vorrichtung (101) nach einem der vorhergehenden Ansprüche, wobei die Vorrichtung ein Durchgangsloch (132) umfasst, wobei Metallschmelze durch das Durchgangsloch (132) hindurchfließen und in einem Behälter (112) unterhalb des Gießlaufs (22) gesammelt werden kann.

11. Verwendung einer Vorrichtung (1) zum Messen der Fließfähigkeit von Metallschmelze (36), wobei die Vorrichtung (1) einen Gießlauf (22) für Metallschmelze (36) umfasst, **dadurch gekennzeichnet, dass** der Gießlauf (22) feuerfeste Fasern umfasst.

## Revendications

1. Dispositif (1) pour mesurer la fluidité de métal fondu (36), dans lequel ledit dispositif (1) comprend une voie de circulation (22) pour le métal fondu (36), et un côté supérieur du dispositif comprend un trou d'entrée pour remplir du métal fondu dans la voie de circulation, **caractérisé en ce que** la voie de circulation (22) comprend une fibre réfractaire.

2. Dispositif (1) selon la revendication 1, dans lequel la voie de circulation (22) comprend une partie supérieure (4) et une partie inférieure (6, 106).

3. Dispositif (1) selon la revendication 2, dans lequel la voie de circulation (22) comprend une partie intermédiaire, dans lequel des parois latérales (26, 126) de la voie de circulation (22, 122) sont formées par la partie intermédiaire.

4. Dispositif (1) selon l'une quelconque des revendications précédentes, comprenant une coupelle (12) pour remplir du métal fondu dans la voie de circulation (22).

5. Dispositif (1) selon la revendication 4, dans lequel la coupelle (12) comprend une fibre réfractaire.

6. Dispositif (1) selon l'une quelconque des revendications 4-5, dans lequel une surface inférieure de la coupelle (12) est formée par une partie supérieure (4) de la face supérieure (8) du dispositif (1).

7. Dispositif (1) selon l'une quelconque des revendications 4-6, dans lequel le dispositif (1) comprend un bouchon (16) pour empêcher du métal fondu de s'écouler de la coupelle (12) jusque dans la voie de circulation (22).

8. Dispositif (1) selon la revendication 7, dans lequel le bouchon (16) comprend un thermomètre pour mesurer la température du métal fondu dans la coupelle (12).

9. Dispositif (1) selon l'une quelconque des revendications précédentes, dans lequel la voie de circulation (22) est fermée et un côté extérieur du dispositif (1) comprend une graduation pour lire la distance de déplacement du métal fondu (36) dans la voie de circulation (22).

10. Dispositif (101) selon l'une quelconque des revendications précédentes, dans lequel le dispositif comprend un trou traversant (132) pour permettre à du métal fondu de s'écouler par le biais du trou traversant (132) et d'être collecté dans un récipient (112) sous la voie de circulation (22).

11. Utilisation d'un dispositif (1) pour mesurer la fluidité de métal fondu (36), dans lequel ledit dispositif (1) comprend une voie de circulation (22) pour du métal fondu (36), **caractérisé en ce que** la voie de circulation (22) comprend une fibre réfractaire.
